# EUROPEAN PATENT SPECIFICATION

(11) **EP 4 092 072 B1**
(45) Date of publication and mention of the grant of the patent: **11.10.2023**
(21) Application number: 22169947.3
(22) Date of filing: 26.04.2022
(51) Int. Cl.: C08J 5/04, C08J 5/06, C08J 3/22

(54) **COMPOSITE RESIN MOLDED BODY HAVING SUSTAINED RELEASE PROPERTY OF MEDICINAL AGENT, AND METHOD FOR MANUFACTURING SAME**
VERBUNDHARZFORMKÖRPER, DER EINE EIGENSCHAFT VON VERZÖGERTER ARZNEIMITTELFREISETZUNG AUFWEIST, UND VERFAHREN ZUR HERSTELLUNG DESSELBEN
CORPS MOULÉ EN RÉSINE COMPOSITE DOTÉ D'UNE PROPRIÉTÉ DE LIBÉRATION PROLONGÉE D'AGENT MÉDICAL, ET SON PROCÉDÉ DE FABRICATION

(30) Priority: 17.05.2021 JP 2021083442
(43) Date of publication of application: 23.11.2022
(73) Proprietor: Panasonic Intellectual Property Management Co., Ltd., Osaka-shi, Osaka 540-6207 (JP)
(72) Inventor: OKABE, Ami, Osaka, 540-6207 (JP); NAGINO, Toshifumi, Osaka, 540-6207 (JP)
(74) Representative: Novagraaf International SA

(56) References cited:
- JP-A- 2019 154 289

## Description

### BACKGROUND

### 1. Technical Field

The present disclosure relates to a composite resin molded body having excellent mechanical characteristics and a sustained release property of a medicinal agent in a humid environment.

### 2. Description of the Related Art

In agricultural applications, pesticides and fertilizer components are used, and in daily necessities, insect repellents, pesticides, fragrances, and deodorant components are used, but these components may be required to be gradually released for a long period of time, that is, to have a so-called sustained release property.

A sustained-release agent is required to have functions such as rigidity that can be applied as a structural member depending on its use and biodegradability that decomposes in a natural environment after sustained-release.

As specific sustained-release agents, those in which a medicinal agent component is carried on a porous base material and those in which a medicinal agent component is dispersed in a degradable material are known.

For example, PTL 1 describes a method in which porous particles are impregnated with an organic solvent to carry a medicinal agent, and surfaces of the porous particles are coated with a polymer. In this case, the polymer coating the porous particles is broken by an external stimulus to release the medicinal agent.

Further, PTL 2 describes a method in which a medicinal agent component is dispersed in a degradable polymer to volatilize the medicinal agent together with decomposition of the polymer. Here, a release rate of the medicinal agent component is controlled by an amount and a decomposition rate of the degradable polymer.

JP 2019 154289 A discloses a fiber composite resin composition allowing the controlled release of a chemical agent, containing a fibrous filler contained in a resin and having a disentanglement site formed in the edge portion of the fibers and a volatile chemical agent carried by the fibrous filler.

### Citation List

### Patent Literature

PTL 1: Unexamined Japanese Patent Publication No. 2009-12996 PTL 2: Unexamined Japanese Patent Publication No. H04-173746

### SUMMARY

A composite resin molded body according to one aspect of the present disclosure is a composite resin molded body containing: a base resin; and a plurality of fibrous fillers dispersed in the base resin, wherein the plurality of fibrous fillers each contain a volatile medicinal agent, when the composite resin molded body is 100 mass%, a content of the plurality of fibrous fillers each containing the volatile medicinal agent in the composite resin molded body is 10 mass% or more and 99 mass% or less, a part of each of at least one of the plurality of fibrous fillers is exposed on a surface of the composite resin molded body, and at least a part of a surface of each of the plurality of fibrous fillers is coated with a hydrolyzable coating resin.

A method of manufacturing a composite resin molded body according to one aspect of the present disclosure includes: a step of preparing a volatile medicinal agent, a fibrous filler, a hydrolyzable coating resin, and a base resin; a step of incorporating the volatile medicinal agent into the fibrous filler; a coating resin melt-kneading step of subjecting the fibrous filler to a melt-kneading treatment together with the hydrolyzable coating resin and promoting fibrillation from an end of the fibrous filler in a fiber length direction to increase a surface area of a defibrated part at the end and to coat at least a part of a surface of the fibrous filler with the hydrolyzable coating resin to form a coated fibrous filler; and a step of kneading the coated fibrous filler together with the base resin and molding a composite resin molded body in which the coated fibrous filler is dispersed in the base resin.

### BRIEF DESCRIPTION OF THE DRAWINGS

Fig. 1 is a schematic cross-sectional view illustrating a cross-sectional structure of a composite resin molded body according to a first exemplary embodiment;
Fig. 2 is a schematic cross-sectional view illustrating a cross-sectional structure of a fibrous filler as a constituent member of the composite resin molded body according to the first exemplary embodiment;
Fig. 3 is a schematic cross-sectional view illustrating a cross-sectional structure of a composite resin molded body containing a fibrous filler having a defibrated site according to the first exemplary embodiment;
Fig. 4 is a schematic diagram of a manufacturing process for the composite resin molded body according to the first exemplary embodiment; and
Fig. 5 is a diagram illustrating configurations and measurement results of composite resin molded bodies in examples and comparative examples in the exemplary embodiment.

### DETAILED DESCRIPTIONS

In PTL 1, since the medicinal agent is completely released at the moment when the polymer covering the porous particles is broken, it is difficult to continuously maintain the efficacy. On the other hand, in PTL 2, the rigidity and durability of the sustained-release agent are limited by the characteristics of the degradable polymer, and therefore the application thereof is limited.

The present disclosure has been made to solve the above-mentioned conventional problems, and an object of the present disclosure is to provide a composite resin molded body that maintains high rigidity during use and has sustained release property of a medicinal agent. The invention is defined in the appended claims.

According to the composite resin molded body according to the present disclosure, compared with a resin alone, it is possible to realize a composite resin molded body having a high elastic modulus and a sustained release property of a medicinal agent in a humid environment.

Hereinafter, a composite resin molded body according to an exemplary embodiment and a method of manufacturing the same will be described with reference to the accompanying drawings. Note that, in the following description, the same components are denoted by the same reference marks, and the description thereof is appropriately omitted.

### (First exemplary embodiment)

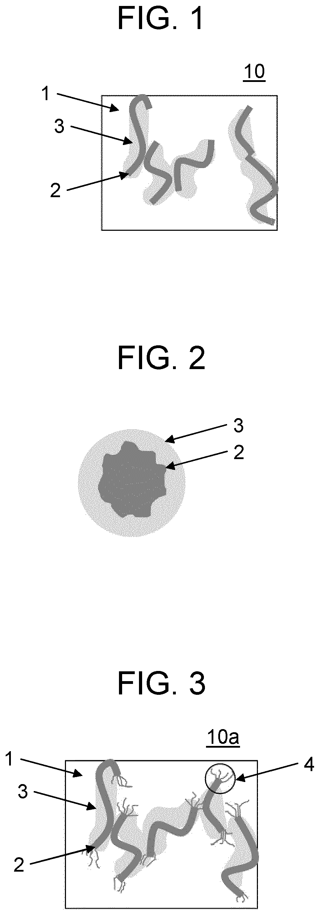

Fig. 1 is a schematic cross-sectional view illustrating a cross-sectional structure of composite resin molded body 10 according to a first exemplary embodiment. Fig. 2 is a schematic cross-sectional view illustrating a cross-sectional structure of fibrous filler 2 that is a constituent member of composite resin molded body 10 according to the first exemplary embodiment.

Composite resin molded body 10 according to the first exemplary embodiment is composed of a melt-kneaded product of base resin 1 and fibrous filler 2 containing a medicinal agent coated with coating resin 3. In composite resin molded body 10, as illustrated in the schematic cross-sectional view of Fig. 1, fibrous filler 2 containing a medicinal agent coated with coating resin 3 is dispersed in base resin 1.

According to composite resin molded body 10, since at least one fibrous filler 2 is exposed on a surface of the molded body, and fibrous fillers 2 have contact points with each other, the composite resin molded body has a high elastic modulus and high water absorbency. In a humid environment, when coating resin 3 is hydrolyzed by water absorption of fibrous filler 2, the medicinal agent contained in fibrous filler 2 is released, and sustained release is promoted through the contact point between fibrous fillers 2. Therefore, it is possible to realize composite resin molded body 10 which maintains high rigidity during use and has sustained release property of a medicinal agent in a humid environment.

Hereinafter, each member constituting the composite resin molded body will be described.

### <Base resin>

In the present exemplary embodiment, base resin 1 is preferably a thermoplastic resin in order to ensure good moldability. Examples of the thermoplastic resin include olefin-based resins (including cyclic olefin-based resins), styrene-based resins, (meth) acrylic resins, organic acid vinyl ester-based resins or derivatives thereof, vinyl ether-based resins, halogen-containing resins, polycarbonate-based resins, polyester-based resins, polyamide-based resins, thermoplastic polyurethane resins, polysulfone-based resins (polyethersulfone, polysulfone, and the like), polyphenylene ether-based resins (such as polymers of 2,6-xylenol), cellulose derivatives (cellulose esters, cellulose carbamates, cellulose ethers, and the like), silicone resins (polydimethylsiloxane, polymethylphenylsiloxane, etc.), rubbers or elastomers (diene rubbers such as polybutadiene and polyisoprene, styrene-butadiene copolymers, acrylonitrile-butadiene copolymers, acrylic rubbers, urethane rubbers, silicone rubbers, and the like), and biomass plastics (polylactic acid, polybutylene succinate, polyhydroxyalkanoic acid, other biodegradable resins, and the like). The above resins may be used alone or in combination of two or more thereof. Note that base resin 1 is not limited to the above materials as long as it has thermoplastic properties.

Among these thermoplastic resins, base resin 1 is preferably an olefin-based resin having a relatively low melting point. Examples of the olefin-based resin include a homopolymer of an olefin-based monomer, a copolymer of an olefin-based monomer, and a copolymer of an olefin-based monomer and another copolymerizable monomer. Examples of the olefin-based monomer include chain olefins (α-C2-20 olefin such as ethylene, propylene, 1-butene, isobutene, 1-pentene, 4-methyl-1-pentene and 1-octene, and the like) and cyclic olefins. These olefin-based monomers may be used alone or in combination of two or more. Among the olefin-based monomers, chain olefins such as ethylene and propylene are preferable. Examples of other copolymerizable monomers include fatty acid vinyl esters such as vinyl acetate and vinyl propionate; (meth) acrylic monomer such as (meth) acrylic acid, alkyl (meth) acrylate, and glycidyl (meth) acrylate; unsaturated dicarboxylic acids such as maleic acid, fumaric acid, maleic anhydride or anhydrides thereof; Vinyl esters of carboxylic acids (for example, vinyl acetate, vinyl propionate, and the like); cyclic olefins such as norbornene and cyclopentadiene; and dienes such as butadiene and isoprene. These copolymerizable monomers may be used alone or in combination of two or more. Specific examples of the olefin-based resin include copolymers of chain olefins (particularly α-C2-4 olefin) such as polyethylene (low density, medium density, high density or linear low density polyethylene, etc.), polypropylene, an ethylene-propylene copolymer, and a terpolymer such as ethylene-propylene-butene-1.

### <Fibrous filler>

Next, fibrous filler 2 will be described. Fibrous filler 2 (Hereinafter, it may be simply referred to as a fiber.) contained in composite resin molded body 10 in the present exemplary embodiment absorbs water in a humid environment. Therefore, the fibrous filler added to the composite resin molded body is used for the main first purpose of bringing coating resin 3 into contact with water to hydrolyze coating resin 3. For this purpose, fibrous filler 2 preferably has high water absorbency, and the moisture content of fibrous filler 2 is preferably 5% or more by a method defined in ASTM D 1909. Specifically, rayon, vinylon, aramid, pulp, cellulose, cellulose nanofiber, lignocellulose, lignocellulose nanofiber, cotton, silk, hemp, chitosan fiber, wool, or the like is preferable.

The second purpose of adding fibrous filler 2 is to improve mechanical characteristics and to improve dimensional stability by decreasing the linear expansion coefficient. For this purpose, fibrous filler 2 preferably has a higher elastic modulus than base resin 1. Specific examples thereof include pulp, cellulose, cellulose nanofibers, lignocellulose, lignocellulose nanofibers, cotton, silk, hemp, chitosan fibers, and wool. Furthermore, among them, celluloses are particularly preferable from the viewpoint of availability, high elastic modulus, and low linear expansion coefficient. Note that fibrous filler 2 is not limited to the above materials as long as it can improve mechanical characteristics and has water absorbency.

The content of medicinal agent-containing fibrous filler 2 in composite resin molded body 10 is preferably 10 mass% or more and 99 mass% or less when composite resin molded body 10 is 100 mass%. When the content of medicinal agent-containing fibrous filler 2 is less than 10 mass%, it is difficult for fibrous fillers 2 to have a contact with each other in composite resin molded body 10, and the composite resin molded body does not have sufficient water absorbency. On the other hand, when the content of medicinal agent-containing fibrous filler 2 is more than 99 mass%, the ratio of base resin 1 and coating resin 3 decreases, so that the effect of bonding fibrous fillers 2 to each other is lost and moldability is deteriorated.

The form of fibrous filler 2 in composite resin molded body 10 will be described. The larger the bonding interface between fibrous filler 2 and coating resin 3 is, the more hydrolysis of coating resin 3 is promoted during water absorption of fibrous filler 2, and therefore the specific surface area of fibrous filler 2 is preferably high. On the other hand, in order to improve the water absorbency of composite resin molded body 10, fibrous filler 2 is preferably exposed on the surface of composite resin molded body 10. Since fibrous filler 2 is exposed on the surface of composite resin molded body 10, water is absorbed from an exposed portion, and water is absorbed into the inside of composite resin molded body 10 by a capillary phenomenon of fibers. The smaller the specific surface area of fibrous filler 2 exposed on the surface of the molded body, the higher the water absorbability. This is because when the specific surface area of fibrous filler 2 exposed on the surface is large, water repellency is enhanced by the effect of fine irregularities. Furthermore, as illustrate in Fig. 3, by having defibrated site 4 at an end of fibrous filler 2, the specific surface area of defibrated site 4 increases, and the number of contact points between fibrous fillers 2 increases, so that the water absorption rate can be increased via the contact points of fibrous fillers 2 in a humid environment.

The central portion of fibrous filler 2 having a small specific surface area and not fibrillated has less entanglement with base resin 1, and is easily exposed to the surface of the molded body depending on the molding conditions. On the other hand, the tip portion of fibrillated fibrous filler 2 is highly entangled with base resin 1, and enters the inside together with base resin 1. As a result, it is possible to obtain composite resin molded body 10 in which the central portion not including both end portions of fibrous filler 2 is exposed to the surface.

Tip defibrated site 4 is preferably 5% or more and 50% or less of fiber length L of entire fibrous filler 2. When the length of defibrated site 4 is less than 5% of total fiber length L, the elastic modulus is not improved because the specific surface area is small, and when the length of defibrated site 4 is more than 50%, defibrated site 4 having a high aspect ratio is exposed on the surface of the molded body, so that water absorbability is deteriorated.

Next, characteristics of fibrous filler 2 will be described. The types of base resin 1 and fibrous fillers 2 are as described above, but when fibrous fillers 2 are too soft, that is, have a small elastic modulus with respect to base resin 1, composite resin molded body 10 has a small elastic modulus as a whole, resulting in a decrease in strength. On the other hand, if fibrous filler 2 is too hard, that is, has a large elastic modulus with respect to base resin 1, shock waves generated at the time of impact are not propagated, and the shock waves are absorbed at the interface between base resin 1 and fibrous filler 2, so that cracks and creases are likely to occur in the vicinity of the interface, and as a result, impact strength is reduced. Therefore, as for the relationship between the elastic modulus of base resin 1 and the elastic modulus of fibrous filler 2, the elastic modulus of fibrous filler 2 is higher, and the difference is preferably as small as possible. The optimum relationship is calculated from simulation results, and the elastic modulus difference between base resin 1 and fibrous filler 2 is preferably within 20 GPa.

Further, these fibrous fillers 2 may be subjected to a surface treatment for the purpose of, for example, improving adhesion to base resin 1 or coating resin 3 or dispersibility in composite resin molded body 10, but when the water absorbency of fibrous fillers 2 is impaired by the surface treatment, it is preferable not to perform the surface treatment in advance.

### <Additive>

Additives may be used as necessary for the purpose of, for example, improving the affinity of base resin 1, coating resin 3, and fibrous filler 2.

### <Coating resin>

Next, coating resin 3 will be described. Coating resin 3 in the present exemplary embodiment is used for the purpose of protecting a medicinal agent contained in fibrous filler 2 and preventing release of the medicinal agent from composite resin molded body 10 during use of composite resin molded body 10. In a humid environment, in order to exhibit sustained release of the medicinal agent, it is necessary that coating resin 3 is decomposed and the medicinal agent contained in fibrous filler 2 is released. Therefore, coating resin 3 is preferably a hydrolyzable resin that decomposes in an environment with a humidity of 50% or more. Specific examples thereof include polyester-based resins such as polylactic acid, polybutylene terephthalate, and polycarbonate. Further, in order to ensure good moldability, a thermoplastic resin is preferable, and the above resins may be used alone or in combination of two or more thereof. Note that coating resin 3 is not limited to the above materials as long as it has hydrolysis characteristics.

Further, in order to maintain a state in which coating resin 3 is coated on at least a part of the surface of fibrous filler 2 in composite resin molded body 10, it is preferable that the melting point of coating resin 3 is higher than or equal to the melting point of base resin 1 and is within a range lower than the carbonization temperature of fibrous filler 2. When the melting point of coating resin 3 is higher than or equal to the melting point of base resin 1, coating resin 3 is not melted during molding of composite resin molded body 10, and when the melting point of coating resin 3 is lower than the carbonization temperature of fibrous filler 2, the coating can be performed without deteriorating fibrous filler 2.

The presence state of coating resin 3 in composite resin molded body 10 will be described. By controlling a coating amount of fibrous filler 2, coating resin 3 can delay the release of the medicinal agent contained in fibrous filler 2 and control a sustained release rate.

### <Medicinal agent>

Next, the medicinal agent will be described. The medicinal agent in the present exemplary embodiment is used as a sustained release component of composite resin molded body 10 in a humid environment. As the medicinal agent in the present exemplary embodiment, insect repellent, insecticide, fragrance, deodorant, pesticide, fertilizer component, and the like can be used according to the purpose, and liquid or solid ones can be used according to other purposes. That is, the type is not particularly limited as long as it can be contained in fibrous filler 2. Specifically, examples of the insect repellent or biorepellent agent include extracts extracted from herbs and spices such as hinokitiol, safrole, limonene, linalool, menthol, 1,8-cineole, citral, eugenol, capsaicin, camphor, vanillin, α-pinene, β-pinene, anethol, anisaldehyde, acetophenone, cinnamyl alcohol, blueleaf alkol, geraniol, naphthalene, diethyltoluamide, lemongrass oil, lemongrass, synthetic musk, pine oil, terpineol, wood vinegar, hexanol, geral formate, γ-lactone, angelica, cyclic terpene alcohol, N, N-diethyl-m-toluamide, ethyl omethone, isothionate, cresol, nonyllaritane, linalool, 2-butoxyethanol, bisether, isophorone, spermine, cinnamic alcohol, methylnonyl ketone, eucalyptol, allyl isothianate, cycloheximide, spearmint oil, orange oil, lemon oil, mandarin oil, lime oil, camphor oil, cassia oil, linaloe oil, peppermint oil, clove oil, pimentin oil, bay oil, fennel oil, anise oil, fennel oil, sogou-flavor, pepper, peppermint, shiso, clove, vanilla, thyme, tangy, rosemary, hamanas, bronsphenes, horseradish, borage, medosweet, glacoumallow, rose geranium, rhubarb, parsley, bergamot, wild strow berry, rocket salad, ginger mint, peppermint, cottage, muscarte, salad barnet, maram, olive, cardoon, pineapple sage, lemon thyme, lemon balm, garden sage, roman camomile, English lavender, lemon bergamot, heart seed, hysop, peny royals, creeping thyme, garden sage, spearmint, bay, greek oregano, and ribwort. Examples of the aromatic or deodorant agent include natural fragrances such as lemon oil, lime oil, spearmint oil, jasmine oil, orange oil, pine oil, tallow oil, lavender oil, and musk oil, or synthetic fragrances using these fragrances as raw materials, for example, limonene, linamol, eugenol, citranerol, vanillin, carboxylic acid, rose oxide, indole, geranyl acetate, and ethyl benzoate.

The above-mentioned medicinal agents may be used alone or in combination of two or more kinds thereof. Note that the volatile medicinal agent is not limited to the above-mentioned materials as long as the volatile medicinal agent can be used for insect repellent, insecticide, aromatic, deodorant, pesticide, fertilizer component, and other purposes.

### <Method of manufacturing composite resin molded body>

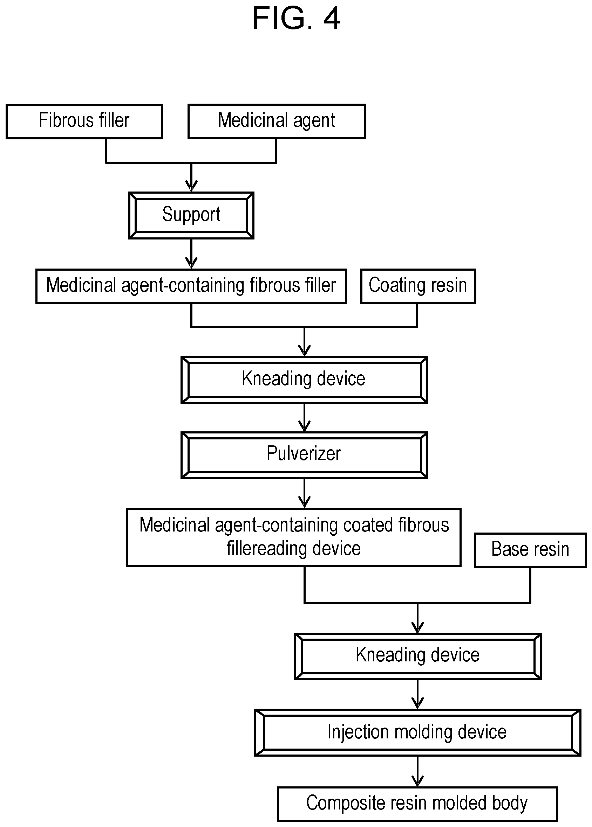

Next, a method of manufacturing composite resin molded body 10 will be described. Fig. 4 is a flowchart illustrating a manufacturing process of composite resin molded body 10 in the present exemplary embodiment.
(1) A medicinal agent is previously supported on fibrous filler 2. The ratio of the medicinal agent to be contained varies depending on the combination of the medicinal agent and the fibrous filler, and examples of a method of supporting the medicinal agent include physical adsorption by dry blending and an impregnation method using a dispersion solvent. The supporting method is not limited to the above as long as it is a method capable of holding the medicinal agent in fibrous filler 2.
(2) Fibrous filler 2 and coating resin 3 are charged into a melt-kneading processing device, and are melt-kneaded in the device. As a result, coating resin 3 is melted, and fibrous filler 2 is dispersed in melted coating resin 3. At the same time, the shearing action of the device promotes fibrillation of aggregates of fibrous fillers 2, and fibrous fillers 2 can be finely dispersed in coating resin 3. At this time, by adjusting the shear conditions, as illustrated in Fig. 3, the end part of fibrous filler 2 is defibrated, and it is also possible to obtain defibrated site 4.

Conventionally, when fibers are combined with a resin, fibers that have been defibrated in advance by a pretreatment such as wet dispersion have been used. However, when the fibrous filler is defibrated in a solvent used in wet dispersion, the fiber swells due to the solvent. Therefore, in order for the fibrous filler to sufficiently absorb water and expand in the composite resin molded body, the solvent in the fibrous filler needs to be dried before being kneaded with the base resin. Further, in the fibrillation by wet dispersion, it is easier to be fibrillated than to be fibrillated in the molten base resin, so that it is difficult to fibrillate only the end part, and the entire fibrous filler is fibrillated. In addition, there is a problem that the number of processes increases and productivity deteriorates by combining pretreatment.

On the other hand, in the process of manufacturing composite resin molded body 10 according to the present exemplary embodiment, a melt-kneading treatment (all-dry method) is performed together with coating resin 3 without performing a pretreatment by wet dispersion for the purpose of fibrillating the fibrous filler. In this method, since the wet dispersion treatment of fibrous filler 2 is not performed, swelling of fibrous filler 2 in the manufacturing process can be suppressed, and the water absorption rate of fibrous filler 2 in composite resin molded body 10 can be improved. By subjecting fibrous filler 2 to a drying treatment in advance or during kneading, the water absorption rate in a humid environment in composite resin molded body 10 can be further improved. When fibrous filler 2 has defibrated site 4 as described above, the fibers have many contact points inside composite resin molded body 10, and the water absorption rate of composite resin molded body 10 can be increased via the contact points between the fibers.

In order to produce fibrous filler 2 of the present exemplary embodiment by the all-dry method, it is preferable to apply high shear stress during kneading, and specific examples of the kneading method include a kneading method using a uniaxial kneader, a biaxial kneader, a roll kneader, a Banbury mixer, and a combination thereof as a kneader. From the viewpoint of easy application of high shear and high mass productivity, a continuous biaxial kneader and a continuous roll kneader are particularly preferable as the kneader. A kneading method other than the above may be used as long as high shear stress can be applied.

(3) The composite resin composition of fibrous filler 2 and coating resin 3 extruded from the melt kneading device is pulverized by a cutting machine or a pulverizer to obtain fibrous filler 2 whose surface is at least partially coated with coating resin 3 in the present exemplary embodiment. Specific examples of the pulverizer include a pulverization method using a pelletizer, a ball mill, a roll mill, a hammer mill, a wonder crusher, a jet pulverizer, and a combination thereof. A pulverization method other than the above may be used as long as the method can maintain a state in which at least a part of fibrous filler 2 is coated with coating resin 3.

(4) An injection-molded article as composite resin molded body 10 can be produced by dry-blending base resin 1 and fibrous filler 2 coated with coating resin 3 and injection-molding them. The molding temperature during injection molding is preferably 125% or less of the melting point of coating resin 3. If the molding temperature is higher than 125% of the melting point of coating resin 3, coating resin 3 is melted and dispersed in base resin 1 during molding.

Hereinafter, examples and comparative examples in experiments performed by the inventors will be described.

### (First example)

In a first example, a cellulose composite polypropylene resin molded body was produced by the following production method.

Softwood pulp (product name: NBKP Celgar, manufactured by Mitsubishi Paper Mills Limited) was used as a starting material for the fibrous filler. As a volatile medicinal agent, limonene (L0047 manufactured by Tokyo Chemical Industry Co., Ltd.) as a citrus fragrance component was used. The softwood pulp was immersed in limonene and milled by a roll mill. Thereafter, excess limonene was removed to obtain a limonene-containing cellulose filler having a mass ratio between the cellulose filler and limonene of 90 : 10.

Polylactic acid (product name: TE-2000, manufactured by UNITIKA LTD.) as a coating resin and the limonene-containing cellulose filler were weighed at a mass ratio of 50 : 50, and dry-blended. Thereafter, the mixture was melt-kneaded with a biaxial kneader (KRC kneader manufactured by Kurimoto, Ltd.). A screw was of a medium shear type. The conditions of the melt-kneading were a material temperature of 200°C and a rotation speed of 50 min⁻¹. The composite resin composition discharged from the biaxial kneader was hot-cut to prepare cellulose composite polylactic acid resin pellets.

The prepared cellulose composite polylactic acid pellets were pulverized by a wonder crusher (WC-3 manufactured by OSAKA CHEMICAL Co., Ltd.) to obtain cellulose fibers coated with a polylactic acid resin. The pulverization condition was set to a rotation speed of 15000 rpm.

Cellulose fibers coated with a polylactic acid resin and a polypropylene resin (manufactured by Prime Polymer Co., Ltd., product name: J108M) as a base resin were weighed at a mass ratio of 50 : 50, and dry-blended. Thereafter, the mixture was melt-kneaded with a biaxial kneader (KRC kneader manufactured by Kurimoto, Ltd.). A screw was of a medium shear type. The conditions of the melt-kneading were a material temperature of 180°C and a rotation speed of 50 min⁻¹. The composite resin composition discharged from the biaxial kneader was hot-cut to produce cellulose composite polypropylene resin pellets in which the mass ratio of the base resin, the medicinal agent-containing fibrous filler and the coating resin was 50 : 25 : 25.

Thereafter, a test piece of the cellulose composite polypropylene resin molded body was produced by an injection molding machine (180AD manufactured by The Japan Steel Works, Ltd.). The preparation conditions of the test piece were a base resin temperature of 200°C, a mold temperature of 50°C, an injection speed of 100 mm/s, and a holding pressure of 100 Pa. A dumbbell-shaped test piece of JIS K7139 type A12 size was produced.

### (Evaluation of water absorbency of fiber)

The water absorption of the fiber was evaluated by measuring the water content of the fiber according to the method defined in ASTM D 1909. Specifically, the weight of the fiber dried at 80°C for 24 hours was measured and taken as a reference weight. Thereafter, the weight of the fiber maintained at a temperature of 20°C and a humidity of 65% for 24 hours was measured. The moisture content was calculated using the weight increase increased from the reference weight as moisture. It was evaluated whether the moisture content was 5% or more. A sample having a moisture content of less than 5% was rated as "N", and a sample having a moisture content of 5% or more was rated as "Y". The moisture content of the softwood pulp was 6.5%, and the evaluation thereof was "Y".

### (Fiber end defibration)

The obtained cellulose composite polypropylene resin molded body was immersed in a chloroform solvent to dissolve polypropylene and polylactic acid, and the shape of the remaining cellulose fibers was observed with a scanning electron microscope. The end parts of the fibers were in a fibrillated state.

### (Evaluation of elastic modulus of composite resin molded body)

A three-point bending test was performed using the obtained test piece. Here, as a method of evaluating the elastic modulus, whether the elastic modulus was 200 MPa or more was determined. A sample having a numerical value of elastic modulus of less than 200 MPa was rated as "N", and a sample having a numerical value of elastic modulus of 200 MPa or more was rated as "Y". The test piece had an elastic modulus of 326 MPa, and the evaluation thereof was "Y".

### (Evaluation of sustained release property of composite resin molded body)

Using the obtained test piece, a sustained release test was performed. A temperature of 60°C and a humidity of 50% were maintained by a hot air dryer, and under the environment, a release duration until the fragrance from the test piece disappeared was measured. For the presence or absence of fragrance, air in the vicinity of the surface of the test piece was measured by an odor measuring device (OMU-Sn manufactured by Futaba Electronics Co., Ltd.) equipped with a light odor sensor, and the time from the start of the measurement until the odor intensity dropped below 1000 was defined as the release duration. As a method of evaluating the sustained release property, it was determined whether the release duration was 500 hours or more. A sample for 500 hours or more was rated "Y", and a sample for less than 500 hours was rated "N". The release duration of the test piece was 720 hours or more, and the evaluation was "Y".

### (Second example)

In a second example, a cellulose composite polypropylene resin molded body was produced in the same manner as in the first example except that the mass ratio of the base resin, the medicinal agent-containing fibrous filler and the coating resin was changed to 67.5 : 25 : 7.5, and the other material conditions and process conditions were the same as those in the first example. The evaluation was performed in the same manner as in the first example.

### (First comparative example)

In a first comparative example, an uncoated medicinal agent-containing cellulose filler was used. A cellulose composite polypropylene resin molded body was produced in the same manner as in the first example except that the mass ratio of the base resin and the medicinal agent-containing cellulose filler was changed to 25 : 75, and the other material conditions and process conditions were the same as those in the first example. The evaluation was performed in the same manner as in the first example.

### (Second comparative example)

In a second comparative example, a medicinal agent-containing PET fiber was produced using a PET fiber having a fiber diameter of 20 µm and a fiber length of 100 µm instead of softwood pulp. A polypropylene composite resin molded body was produced in the same manner as in the first example under other material conditions and process conditions. The evaluation was performed in the same manner as in the first example.

### (Third comparative example)

In a third comparative example, a coating resin and a medicinal agent were melt-kneaded at a mass ratio of 95 : 5 without using a fibrous filler. A polypropylene composite resin molded body was produced in the same manner as in the first example except that the mass ratio of the base resin and the medicinal agent-containing polylactic acid resin was changed to 50 : 50, and the other material conditions and process conditions were the same as those in the first example. The evaluation was performed in the same manner as in the first example.

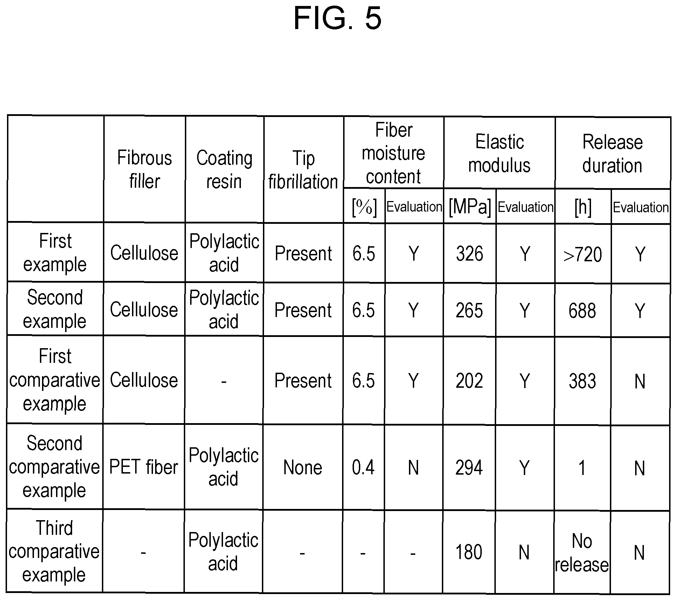

Fig. 5 illustrates the configuration and measurement results of the composite resin molded bodies in the first and second examples and the first to the third comparative examples.

As is apparent from Fig. 5, in the first and second examples in which the medicinal agent contained in the fibrous filler was protected by the coating resin, the elastic modulus was as high as 200 MPa or more, and in the sustained release test in a humid environment, sustained release was confirmed as compared with the third comparative example. In addition, in the first example in which the ratio of the coating resin was larger than that in the second example, sustained release for a long period was exhibited.

It was confirmed that a composite resin having a high elastic modulus and sustained-release properties of a medicinal agent was obtained when a fibrous filler containing a volatile medicinal agent was compounded, the fibrous filler was at least partially covered with a hydrolyzable resin on the fiber surface, the fiber was exposed on the surface of the composite resin molded body, and the water absorption rate of the fibrous filler was high.

In the first comparative example produced without using a coating resin, the elastic modulus was improved as compared with the third comparative example by combining the fibrous fillers. However, since the medicinal agent was not protected by the coating resin, the sustained release time was reduced as compared with the first example, and the evaluation was "N".

In the second comparative example produced using PET fibers as the fibrous fillers, hydrolysis of the polylactic acid resin did not proceed in the evaluation of sustained release, because the moisture content of the PET fibers was low and water absorbability was absent, and the release duration time was reduced as compared with the first example, and the evaluation was "N".

In the third comparative example prepared without using the fibrous filler, the sustained release property was not exhibited, and the sustained release property evaluation was "N".

From the above evaluation, it was confirmed that when a volatile medicinal agent is contained in the fibrous filler having water absorbency, at least a part of the surface of the fiber is coated with a hydrolyzable coating resin, and the fiber is exposed on the surface of the composite resin molded body, a composite resin molded body having a high elastic modulus and sustained release of the medicinal agent is obtained.

Note that the present disclosure includes appropriate combination of arbitrary exemplary embodiments and/or examples among the various exemplary embodiments and/or examples described above, and effects of the respective exemplary embodiments and/or examples can be exhibited.

According to the composite resin molded body according to an aspect of the present disclosure, it is possible to provide a fiber composite resin molded body having sustained release of a medicinal agent component, which has both sustainability of release of a pest control or insecticidal component, a fragrance or deodorizing component, an agrochemical or fertilizer component, and other medicinal agent components, and high rigidity applicable to structural members.

## Claims

1. A composite resin molded body comprising:
a base resin; and
a plurality of fibrous fillers dispersed in the base resin,
wherein the plurality of fibrous fillers each contain a volatile medicinal agent,
when the composite resin molded body is 100 mass%, a content of the plurality of fibrous fillers each containing the volatile medicinal agent in the composite resin molded body is 10 mass% or more and 99 mass% or less,
a part of each of at least one of the plurality of fibrous fillers is exposed on a surface of the composite resin molded body, and
at least a part of a surface of each of the plurality of fibrous fillers is coated with a hydrolyzable coating resin.

2. The composite resin molded body according to Claim 1, wherein a moisture content of the plurality of fibrous fillers is 5% or more by a method defined by ASTM D 1909.

3. The composite resin molded body according to Claim 1 or 2, wherein the hydrolyzable coating resin has a melting point higher than or equal to a melting point of the base resin and within a range lower than a carbonization temperature of the plurality of fibrous fillers.

4. The composite resin molded body according to any one of Claims 1 to 3,
wherein the plurality of fibrous fillers are celluloses.

5. The composite resin molded body according to any one of Claims 1 to 4,
wherein at least one of the plurality of fibrous fillers has a defibrated site formed at an end in a fiber length direction.

6. A method of manufacturing a composite resin molded body, comprising:
a step of preparing a volatile medicinal agent, a fibrous filler, a hydrolyzable coating resin, and a base resin;
a step of incorporating the volatile medicinal agent into the fibrous filler;
a coating resin melt-kneading step of subjecting the fibrous filler to a melt-kneading treatment together with the hydrolyzable coating resin and promoting fibrillation from an end of the fibrous filler in a fiber length direction to increase a surface area of a defibrated part at the end and to coat at least a part of a surface of the fibrous filler with the hydrolyzable coating resin to form a coated fibrous filler; and
a step of kneading the coated fibrous filler together with the base resin and molding a composite resin molded body in which the coated fibrous filler is dispersed in the base resin.

7. The method according to Claim 6, wherein in the step of molding the composite resin molded body, a molding temperature is set to a temperature of 125% or less of a melting point of the hydrolyzable coating resin.

## Patentansprüche

1. Verbundharz-Formteil, umfassend:
ein Grundharz und
mehrere Faserfüllstoffe, die in dem Grundharz dispergiert sind,
wobei die mehreren Faserfüllstoffe jeweils einen flüchtigen Arzneistoff enthalten,
das Verbundharz-Formteil 100 Massen-% ausmacht, wobei ein Gehalt der mehreren Faserfüllstoffe, die jeweils den flüchtigen Arzneistoff enthalten, in dem Verbundharz-Formteil 10 Massen-% oder mehr und 99 Massen-% oder weniger beträgt,
ein Teil jedes von mindestens einem von den mehreren Faserfüllstoffen auf einer Oberfläche des Verbundharz-Formteils freigelegt ist und
mindestens ein Teil einer Oberfläche jedes von den mehreren Faserfüllstoffen mit einem hydrolysierbaren Beschichtungsharz beschichtet ist.

2. Verbundharz-Formteil nach Anspruch 1, wobei ein Feuchtigkeitsgehalt der mehreren Faserfüllstoffe anhand eines Verfahrens, das durch ASTM D 1909 definiert ist, 5 % oder mehr beträgt.

3. Verbundharz-Formteil nach Anspruch 1 oder 2, wobei das hydrolysierbare Beschichtungsharz einen Schmelzpunkt aufweist, der höher als oder gleich einem Schmelzpunkt des Grundharzes ist und innerhalb eines Bereichs liegt, der niedriger als eine Karbonisierungstemperatur der mehreren Faserfüllstoffe ist.

4. Verbundharz-Formteil nach einem der Ansprüche 1 bis 3, wobei die mehreren Faserfüllstoffe Cellulosen sind.

5. Verbundharz-Formteil nach einem der Ansprüche 1 bis 4, wobei mindestens einer von den mehreren Faserfüllstoffen eine zerfaserte Stelle aufweist, die an einem Ende in einer Faserlängenrichtung ausgebildet ist.

6. Verfahren zur Herstellung eines Verbundharz-Formteils, umfassend:
einen Schritt eines Vorbereitens eines flüchtigen Arzneistoffs, eines Faserfüllstoffs, eines hydrolysierbaren Beschichtungsharzes und eines Grundharzes;
einen Schritt eines Integrierens des flüchtigen Arzneistoffs in den Faserfüllstoff;
einen Beschichtungsharzknetschritt eines Unterziehens des Faserfüllstoffs einer Schmelzknetbehandlung zusammen mit dem hydrolysierbaren Beschichtungsharz und eines Förderns einer Faserung von einem Ende des Faserfüllstoffs in einer Faserlängenrichtung, um einen Oberflächenbereich eines zerfaserten Teils an dem Ende zu erhöhen und mindestens einen Teil einer Oberfläche des Faserfüllstoffs mit dem hydrolysierbaren Beschichtungsharz zu beschichten, um einen beschichteten Faserfüllstoff zu bilden, und
einen Schritt eines Knetens des beschichteten Faserfüllstoffs zusammen mit dem Grundharz und Formen eines Verbundharz-Formteils, in dem der beschichtete Faserfüllstoff in dem Grundharz dispergiert ist.

7. Verfahren nach Anspruch 6, wobei eine Formungstemperatur in dem Schritt des Formens des Verbundharz-Formteils auf eine Temperatur von 125 % oder weniger eines Schmelzpunkts des hydrolysierbaren Beschichtungsharzes eingestellt ist.

## Revendications

1. Corps moulé en résine comprenant :
une résine de base ; et
une pluralité de charges fibreuses dispersées dans la résine de base,
dans lequel chacune parmi la pluralité de charges fibreuses contient un agent médicinal volatil,
quand le corps moulé en résine composite représente 100 % en masse, la teneur en la pluralité de charges fibreuses contenant chacune l'agent médicinal volatil dans le corps moulé en résine composite est de 10 % en masse ou plus et 99 % en masse ou moins,
une partie de chacune parmi au moins une de la pluralité de charges fibreuses est exposée sur une surface du corps moulé en résine composite, et
au moins une partie d'une surface de chacune parmi la pluralité de charges fibreuses est revêtue d'une résine de revêtement hydrolysable.

2. Corps moulé en résine composite selon la revendication 1, dans lequel la teneur en humidité de la pluralité de charges fibreuses est de 5 % ou plus par un procédé défini par la norme ASTM D 1909.

3. Corps moulé en résine composite selon la revendication 1 ou 2, dans lequel la résine de revêtement hydrolysable a un point de fusion supérieur ou égal au point de fusion de la résine de base et situé dans une plage inférieure à la température de carbonisation de la pluralité de charges fibreuses.

4. Corps moulé en résine composite selon l'une quelconque des revendications 1 à 3, dans lequel la pluralité de charges fibreuses consiste en celluloses.

5. Corps moulé en résine composite selon l'une quelconque des revendications 1 à 4, dans lequel au moins une parmi la pluralité de charges fibreuses a un site défibré formé à une extrémité dans la direction de la longueur des fibres.

6. Procédé de fabrication d'un corps moulé en résine composite, comprenant :
une étape de préparation d'un agent médicinal volatil, d'une charge fibreuse, d'une résine de revêtement hydrolysable, et d'une résine de base ;
une étape d'incorporation de l'agent médicinal volatil dans la charge fibreuse ;
une étape de malaxage à l'état fondu de la résine de revêtement dans laquelle la charge fibreuse est soumise à un traitement de malaxage à l'état fondu conjointement avec la résine de revêtement hydrolysable et une fibrillation depuis une extrémité de la charge fibreuse dans la direction de la longueur des fibres est favorisée pour que la superficie de la partie défibrée à l'extrémité soit augmentée et pour qu'au moins une partie de la surface de la charge fibreuse soit revêtue de la résine de revêtement hydrolysable pour former une charge fibreuse revêtue ; et
une étape de malaxage de la charge fibreuse revêtue conjointement avec la résine de base et de moulage d'un corps moulé en résine composite dans lequel la charge fibreuse revêtue est dispersée dans la résine de base.

7. Procédé selon la revendication 6, dans lequel, dans l'étape de moulage du corps moulé en résine composite, la température de moulage est établie à une température égale à 125 % ou moins du point de fusion de la résine de revêtement hydrolysable.
